(19) Europäisches Patentamt
European Patent Office
Office européen des brevets

(11) **EP 3 733 122 A1**

(12) **EUROPEAN PATENT APPLICATION**
published in accordance with Art. 153(4) EPC

(43) Date of publication:
**04.11.2020 Bulletin 2020/45**

(21) Application number: **18894225.4**

(22) Date of filing: **12.12.2018**

(51) Int Cl.:
*A61F 2/07* (2013.01)    *A61L 31/06* (2006.01)
*A61L 31/12* (2006.01)    *A61L 31/14* (2006.01)
*D03D 1/00* (2006.01)    *D03D 3/02* (2006.01)

(86) International application number:
**PCT/JP2018/045747**

(87) International publication number:
**WO 2019/131148 (04.07.2019 Gazette 2019/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2017 JP 2017254737**

(71) Applicant: **Asahi Kasei Kabushiki Kaisha
Tokyo 1000006 (JP)**

(72) Inventors:
• **OKUNO, Tokio**
  **Tokyo 100-0006 (JP)**
• **FUKUDA, Ryo**
  **Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **MEDICAL FABRIC**

(57)    Provided is a high-density medical fabric that can be suitably used as a graft for a branched stent graft, can accommodate diameter changes, has the burst strength required of a material to be implanted in a body, and has a seamless tubular shape that can narrow in diameter. This high-density medical fabric is characterized by satisfying the conditions (1), (2), (3), and (4). (1) Both warp and weft yarns are synthetic multifilament fibers having a total fiber fineness of 60 dtex or less. (2) The fabric is provided with a large-diameter part, two branch parts having summed diameters smaller than the diameter of the large-diameter part, and a tapered part interposed between the large-diameter part and the two branch parts, the large-diameter part having a 1/1 plain woven structure, the two branch parts having, for example, a 1/2 ribbed woven structure, and the tapered part having a structure in which these woven structures alternate to form a triangle shape. (3) In each of the large-diameter part and the two branch parts, the fabric has a cover factor of 1600 to 2400. (4) In each of the large-diameter part and the two branch parts, the fabric has a thickness of 110 μm or less.

FIG. 1

Large-diameter portion (A)

Tapered portion

Branch portions (B)

1/1 Plain

1/2 Rib

The gray portions in the left panel have the 1/1 plain-1/2 rib combination illustrated above (one-yarn-alternating structure)

## Description

FIELD

The present invention relates to a medical high density woven fabric. More specifically, the present invention relates to a seamless tubular medical high density woven fabric which has low thickness, high strength, and low water permeability, and which enables reduction of the diameter, the woven fabric including: a large-diameter portion; two branch portions having diameters whose sum is larger or smaller than the diameter of the large-diameter portion; and a tapered portion arranged between the large-diameter portion and the two branch portions; the tapered portion having a particular structure showing a shift in the woven structure so as to conform to the diameter change, and also relates to a stent graft prepared by suturing and fixing a metal stent to the inner face and/or outer face of the woven fabric using a suture thread, to use the woven fabric as a graft.

BACKGROUND

Thanks to the recent progress of medical technologies, the therapeutic method for aortic aneurysms is being rapidly replaced from artificial blood vessel replacement to stent graft operation, which is less invasive. Conventional artificial blood vessel replacement requires an extensive surgical operation involving thoracotomy or laparotomy, which imposes a heavy burden on the patient. Therefore, its application to elderly patients and patients with comorbidities is limited, and furthermore, it requires long-term hospitalization and hence imposes a heavy economic burden on the patient and the medical facility, which is problematic. In contrast, application of the stent graft operation has been rapidly increasing in recent years since transcatheter endovascular treatment (a therapeutic method in which a thin catheter containing a stent graft compressively inserted therein is introduced through the artery at the base of a leg, which stent graft is then opened and fixed at the site of the aneurysm to block blood flow into the aneurysm, to thereby prevent the aneurysm from rupturing) using a stent graft, which contains a graft such as a medical woven fabric or membrane having a tubular shape combined with a stent that plays a role in maintaining the tubular shape with a metal, does not involve thoracotomy or laparotomy, and therefore the physical and economic burdens can be reduced.

However, as described in PTL 1, the current stent grafts use a stent having a large metal wire diameter and a graft with high thickness, and hence cannot be folded into a small diameter. Thus, they always have a large catheter diameter, and are often not applicable to females and Asians such as Japanese having thin arteries. Thinning of a stent graft requires modification of the shape of the metal stent, the metal wire diameter, and/or the like. However, since fixation of a stent graft to an affected area is basically based on a method in which the stent graft is pressed against the vascular wall utilizing the expansive force of the metal, there is a limitation in the improvement when it affects the expansive force, such as when the stent wire diameter is reduced. On the other hand, thinning of the graft, which occupies a large part of the volume of the stent graft, has also been demanded. However, when, for example, the thickness of an e-PTFE membrane is reduced, the membrane may be stretched to become thinner with time due to the expansive force applied by the stent and the blood pressure, leading to bursting. In view of this, PTL 1 proposes use of a superfine polyester fiber having both high biological safety and moldability.

As described in PTL 2, when the graft is made of a woven fabric composed of fibers, or made of a knitted fabric, the thinning causes blood leakage from the graft itself, which prevents the therapeutic effect. In particular, branched stent grafts used for the treatment of abdominal aortic aneurysms tend to cause leakage from the boundary portion at which the aorta is branched to the lower limbs (left and right iliac arteries), and this problem becomes more obvious as the thickness decreases. Moreover, the branched portion (boundary portion) tends to receive an extension or bending stress, which may cause rupture of a membrane-type graft. In woven fabric-type grafts, the boundary portion is hand-sewn, or the end face treatment is carried out using a thermal cutter, to prevent blood leakage or rupture at the boundary portion site. However, these countermeasures are still insufficient. In view of this, for achieving both the prevention of leakage from the branched portion (boundary portion) and the reduction of the diameter, PTL 2 proposes a seamless tubular medical high density woven fabric using a polyester multifilament yarn having a monofilament fineness of not more than 0.5 dtex as the weft yarn, wherein the woven texture of the branched portion (boundary portion) is constituted by a single texture.

PTL 3 describes that, in a graft material woven using an ordinary dobby loom, for enabling tapering in a tapered portion provided between a single lumen upper portion of a woven graft trunk and two small-diameter lumens, pick (weft) yarns are interwoven around every two warp yarns to enable a tight, relatively impermeable weave for the upper portion of the trunk, and to enable more dense "packing" of waves for the tapered portion and the two branch portions (see paragraph [0058] of the literature).

However, PTL 3 does not describe at all that the densely "packed" woven texture is a particular woven structure, i.e., a 1/2 rib woven structure, or a 1/1 plain-1/2 rib one-yarn-alternating or two-yarn-alternating woven structure, and does not describe at all that, in the tapered portion, in one front view, shifting from the woven structure of the trunk to the woven structure of the branch portions occurs to form a triangular shape from a certain point on the line in contact with the trunk toward two points on the line in contact with the two branch portions. Further, the woven fabric for the graft material described in PTL 3 does not use a polyester multifilament yarn having a monofilament fineness of not more than 0.5 dtex as the weft yarn.

As described above, the seamless tubular medical high density woven fabric described in PTL 1 and/or PTL 2 uses a polyester multifilament yarn having a monofilament fineness of not more than 0.5 dtex as the weft yarn. Therefore, the woven fabric can reduce the thickness of the graft, and can achieve reduction of the diameter of the stent graft while maintaining the required low water permeability, high burst strength, and thinness. However, these literatures do not disclose, teach, or suggest how, when the woven fabric includes: a large-diameter portion; two branch portions having diameters whose sum is larger or smaller than the diameter of the large-diameter portion; and a tapered portion arranged between the large-diameter portion and the two branch portions; shifting between the woven structures in the tapered portion should occur to conform to the diameter change.

[CITATION LIST]

[PATENT LITERATURE]

[PTL 1] WO 2013/137263
[PTL 2] Japanese Unexamined Patent Publication (Kokai) No. 2016-123764
[PTL 3] Japanese Patent Publication No. 4540912

SUMMARY

[TECHNICAL PROBLEM]

In view of the problems in the conventional techniques, an object of the present invention is to provide a seamless tubular medical high density woven fabric which can be suitably used as a graft for a branched stent graft, which conforms to a diameter change, which has the burst strength required for a material to be implanted in the body, and which enables reduction of the diameter.

[SOLUTION TO PROBLEM]

As a result of intensive study and experiments, the present inventors discovered that, when a synthetic polyester multifilament fiber having a total fineness of not more than 60 dtex is used both as the warp yarn and the weft yarn, and where, at the same time, the woven fabric includes: a large-diameter portion; two branch portions having diameters whose sum is larger or smaller than the diameter of the large-diameter portion; and a tapered portion arranged between the large-diameter portion and the two branch portions; the above problems can be solved by providing the tapered portion such that it has a particular structure showing a shift in the woven structure so as to conform to the diameter change, thereby completing the present invention.
More specifically, the present invention is as follows.

[1] A seamless tubular medical high density woven fabric, satisfying the following (1) to (4):

(1) both warp yarn and weft yarn are synthetic multifilament fibers having a total fineness of not more than 60 dtex;
(2) the woven fabric comprises: a large-diameter portion; two branch portions having diameters whose sum is larger or smaller than the diameter of the large-diameter portion; and a tapered portion arranged between the large-diameter portion and the two branch portions;
wherein, when the sum of the diameters of the two branch portions is smaller than the diameter of the large-diameter portion, the large-diameter portion has a 1/1 plain woven structure; the two branch portions have a 1/2 rib woven structure, or have a 1/1 plain-1/2 rib one-yarn-alternating or two-yarn-alternating woven structure; the tapered portion is bounded, in one front view, by a line in contact with the large-diameter portion, and a line in contact with the two branch portions; and shifting from the woven structure of the large-diameter portion to the woven structure of the two branch portions occurs to form a triangular shape

from the foot of the line extending, from the meeting point of the two branch portions on the line in contact with the two branch portions, toward, and perpendicularly crossing with, the line in contact with the large-diameter portion,

toward two arbitrary points on the line in contact with the two branch portions; and

wherein, when the sum of the diameters of the two branch portions is larger than the diameter of the large-diameter portion, the large-diameter portion has a 1/2 rib woven structure, or has a 1/1 plain-1/2 rib one-yarn-alternating or two-yarn-alternating woven structure; the two branch portions have a 1/1 plain woven structure; the tapered portion is bounded, in one front view, by a line in contact with the large-diameter portion, and a line in contact with the two branch portions; and shifting from the woven texture of the two branch portions to the woven structure of the large-diameter portion occurs to form a triangular shape from the meeting point of the two branch portions on the line in contact with the two branch portions toward two arbitrary points on the line in contact with the large-diameter portion;

(3) the woven fabric in each of the large-diameter portion and the two branch portions has a cover factor of 1600 to 2400; and

(4) the thickness of the woven fabric in each of the large-diameter portion and the two branch portions is not more than 110 $\mu$m.

[2] The medical high density woven fabric according to [1], wherein the woven fabric in the tapered portion also has a cover factor of 1600 to 2400.

[3] The medical high density woven fabric according to [2], wherein the woven fabric in each of the large-diameter portion, the two branch portions, and the tapered portion has a water permeability of not more than 500 ml/cm$^2$/min.

[4] The medical high density woven fabric according to any one of [1] to [3], wherein the weft yarn is a synthetic polyester multifilament fiber having a monofilament fineness of not more than 0.5 dtex.

[5] The medical high density woven fabric according to any one of [1] to [4], wherein the woven fabric in each of the large-diameter portion, the two branch portions, and the tapered portion has a burst strength of not less than 100 N.

[ADVANTAGEOUS EFFECTS OF INVENTION]

The seamless tubular medical high density woven fabric according to the present invention has low thickness and high strength, and enables reduction of the diameter. Further, when the woven fabric includes: a large-diameter portion; two branch portions having diameters whose sum is larger or smaller than the diameter of the large-diameter portion; and a tapered portion arranged between the large-diameter portion and the two branch portions; the tapered portion can have a particular structure showing a shift in the woven texture so as to conform to the diameter change. Thus, the woven fabric is useful as a graft for a stent graft in which the graft is sutured and fixed to a metal stent using a suture thread.

BRIEF DESCRIPTION OF DRAWINGS

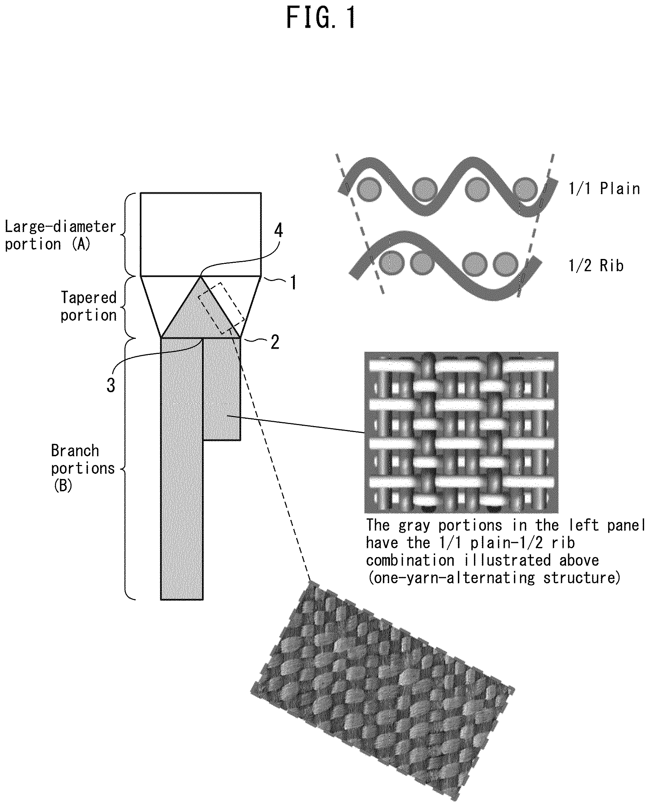

FIG. 1 is a front view illustrating a branched graft in the present embodiment including a large-diameter portion (body), two branch portions (legs), and a tapered portion. The large-diameter portion and part of the tapered portion have a 1/1 plain woven structure. The gray portions, corresponding to the branch portions and part of the tapered portion, have a 1/1 plain-1/2 rib one-yarn-alternating woven structure. In the figure, "1" represents a line in contact with the large-diameter portion; "2" represents a line in contact with the branch portions; "3" represents a meeting point of the two branch portions; and "4" represents an intersection point (foot) between a line perpendicularly drawn from the meeting point and "1".

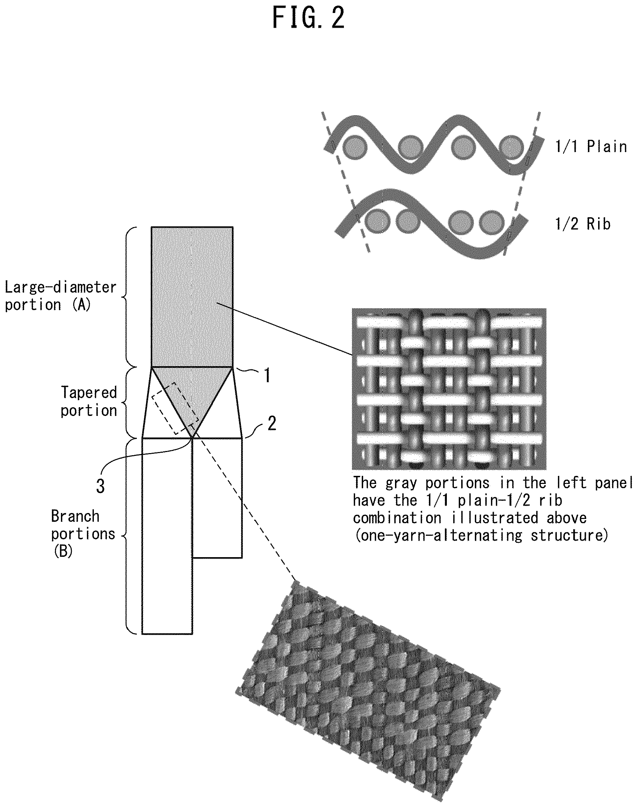

FIG. 2 is a front view illustrating a branched graft in the present embodiment including a large-diameter portion (body), two branch portions (legs), and a tapered portion. The gray portions, corresponding to the large-diameter portion and part of the tapered portion, have a 1/1 plain-1/2 rib one-yarn-alternating woven structure. The branch portions and part of the tapered portion have a 1/1 plain woven structure. In the figure, "1" represents a line in contact with the large-diameter portion; "2" represents a line in contact with the branch portions; and "3" represents a meeting point of the two branch portions.

FIG. 3 is a woven structure for a case where the warp and weft in the front side of a double-woven structure form a 1/1 plain structure, and illustrates a woven structure designed only for the warp and weft in the front side, and a 3D schematic diagram thereof.

FIG. 4 is a woven structure for a case where the warp and weft in both the front side and the back side of a double-woven structure form a 1/1 plain structure, and illustrates a woven structure designed for the double weaving, and a 3D schematic diagram thereof.

FIG. 5 is a woven texture for a case where the warp and weft in the front side of a double-woven structure form a 1/2 rib structure, and illustrates a woven structure designed only for the warp and weft in the front side, and a 3D schematic diagram thereof.

FIG. 6 is a woven texture for a case where the warp and weft in both the front side and the back side of a double-woven structure form a 1/2 rib structure, and illustrates a woven structure designed for the double weaving, and a 3D schematic diagram thereof.

FIG. 7 is a woven structure for a case where the warp and weft in the front side of a double-woven structure form a 1/1 plain-1/2 rib one-yarn-alternating woven structure, and illustrates a woven structure designed only for the warp and weft in the front side, and a 3D schematic diagram thereof.

FIG. 8 is a woven structure for a case where the warp and weft in both the front side and the back side of a double-woven structure form a 1/1 plain-1/2 rib one-yarn-alternating woven structure, and illustrates a woven structure designed for the double weaving, and a 3D schematic diagram thereof.

FIG. 9 is a woven structure for a case where the warp and weft in the front side of a double-woven structure form a 1/1 plain-1/2 rib two-yarn-alternating woven structure, and illustrates a woven structure designed only for the warp and weft in the front side, and a 3D schematic diagram thereof.

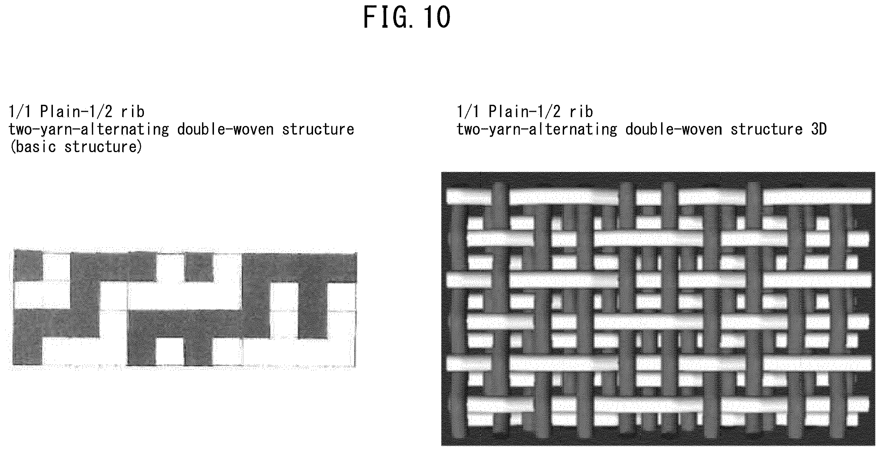

FIG. 10 is a woven structure for a case where the warp and weft in both the front side and the back side of a double-woven structure form a 1/1 plain-1/2 rib two-yarn-alternating woven structure, and illustrates a woven texture designed for the double weaving, and a 3D schematic diagram thereof.

FIG. 11 is drawing-substituting photographs of a branched graft after post-processing using a heat setting bar.

DESCRIPTION OF EMBODIMENTS

An embodiment of the present invention is described below in detail.

The medical high density woven fabric of the present embodiment is a seamless tubular medical high density woven fabric satisfying the following (1) to (4):

(1) both warp yarn and weft yarn are synthetic multifilament fibers having a total fineness of not more than 60 dtex;
(2) the woven fabric comprises: a large-diameter portion; two branch portions having diameters whose sum is larger or smaller than the diameter of the large-diameter portion; and a tapered portion arranged between the large-diameter portion and the two branch portions;
wherein, when the sum of the diameters of the two branch portions is smaller than the diameter of the large-diameter portion, the large-diameter portion has a 1/1 plain woven structure; the two branch portions have a 1/2 rib woven structure, or have a 1/1 plain-1/2 rib one-yarn-alternating or two-yarn-alternating woven structure; the tapered portion is bounded, in one front view, by a line in contact with the large-diameter portion, and a line in contact with the two branch portions; and shifting from the woven structure of the large-diameter portion to the woven structure of the two branch portions occurs to form a triangular shape

from the foot of the line extending, from the meeting point of the two branch portions on the line in contact with the two branch portions, toward, and perpendicularly crossing with, the line in contact with the large-diameter portion,
toward two arbitrary points on the line in contact with the two branch portions; and
wherein, when the sum of the diameters of the two branch portions is larger than the diameter of the large-diameter portion, the large-diameter portion has a 1/2 rib woven structure, or has a 1/1 plain-1/2 rib one-yarn-alternating or two-yarn-alternating woven structure; the two branch portions have a 1/1 plain woven structure; the tapered portion is bounded, in one front view, by a line in contact with the large-diameter portion, and a line in contact with the two branch portions; and shifting from the woven structure of the two branch portions to the woven texture of the large-diameter portion occurs to form a triangular shape
from the meeting point of the two branch portions on the line in contact with the two branch portions
toward two arbitrary points on the line in contact with the large-diameter portion;

(3) the woven fabric in each of the large-diameter portion and the two branch portions has a cover factor of 1600 to 2400; and
(4) the thickness of the woven fabric in each of the large-diameter portion and the two branch portions is not more than 110 $\mu$m.

In the present description, the term "seamless" means that each warp yarn constituting a tubular woven fabric is continuous in the longitudinal direction, and that the weft yarns continue in the weft-yarn direction to form a woven structure.

Both warp yarn and weft yarn constituting (removed from) the seamless tubular medical high density woven fabric of the present embodiment are synthetic multifilament fibers having a total fineness of not more than 60 dtex. The total fineness is preferably 7 dtex to 60 dtex from the viewpoint of thinness and strength of the woven fabric for a stent graft. When the total fineness is not less than 7 dtex, a tensile strength can be secured even with the thinness of the woven fabric, so that the woven fabric meets the demand for reduction of the diameter of the stent graft. When the total fineness is not more than 60 dtex, the woven fabric is not too thick, and suitable for the reduction of the diameter. From the viewpoint of satisfying both the thinness and the practical performance of the woven fabric, the total fineness is more preferably 10 dtex to 55 dtex, still more preferably 15 dtex to 50 dtex.

When the thickness of the woven fabric is not more than 110 $\mu$m, when a tubular woven fabric having an inner diameter of 40 mm is prepared, it can pass through a hole having a diameter of 6 mm (assuming a catheter having an inner diameter of 6 mm).

The weft yarn constituting the woven fabric of the present embodiment is preferably a superfine fiber having a monofilament fineness of not more than 0.5 dtex. When the monofilament fineness is not more than 0.5 dtex, affinity with vascular endothelial cells increases to promote integration of the woven fabric with the vascular wall tissue, so that prevention of movement or detachment of the stent graft in the blood vessel, and suppression of thrombogenesis can be expected. From the viewpoint of thinness and cell affinity of the woven fabric, the fiber has a monofilament fineness of more preferably not more than 0.4 dtex, still more preferably not more than 0.3 dtex, still more preferably not more than 0.2 dtex. There is no lower limit of the monofilament fineness. From the viewpoint of the processability during the warping, weaving, and the like in the woven fabric production process, and achievement of the burst strength of the woven fabric, the monofilament fineness is preferably not less than 0.01 dtex, more preferably not less than 0.03 dtex.

The warp yarn constituting the woven fabric of the present embodiment has a monofilament fineness of preferably not less than 1.0 dtex, more preferably not less than 1.3 dtex, still more preferably not less than 1.4 dtex. When the warp yarn has a monofilament fineness of not less than 1.0 dtex, the warp yarn can maintain a higher tensile strength compared to the superfine fiber that is the weft yarn; handling during the weaving can be made easier; and the shape stability as a tubular woven fabric can be improved.

The tubular woven fabric of the present embodiment includes: a large-diameter portion; two branch portions having diameters whose sum is larger or smaller than the diameter of the large-diameter portion; and a tapered portion arranged between the large-diameter portion and the two branch portions;

wherein, when the sum of the diameters of the two branch portions is smaller than the diameter of the large-diameter portion, the tapered portion is bounded by a line in contact with the large-diameter portion, and a line in contact with the two branch portions; and shifting from the woven texture of the large-diameter portion to the woven texture of the two branch portions occurs to form a triangular shape

from the foot of the line extending, from the meeting point of the two branch portions on the line in contact with the two branch portions, toward, and perpendicularly crossing with, the line in contact with the large-diameter portion, toward two arbitrary points on the line in contact with the two branch portions. Alternatively, when the sum of the diameters of the two branch portions is larger than the diameter of the large-diameter portion, the large-diameter portion has a 1/2 rib woven structure, or has a 1/1 plain-1/2 rib one-yarn-alternating or two-yarn-alternating woven structure; the two branch portions have a 1/1 plain woven structure; the tapered portion is bounded, in one front view, by a line in contact with the large-diameter portion, and a line in contact with the two branch portions; and shifting from the woven structure of the two branch portions to the woven structure of the large-diameter portion occurs to form a triangular shape

from the meeting point of the two branch portions on the line in contact with the two branch portions

toward two arbitrary points on the line in contact with the large-diameter portion.

As illustrated in FIGs. 1 and 2, the "line (1) in contact with the large-diameter portion" means the border between the large-diameter portion (A) and the tapered portion, in which the diameter change occurs; the "line (2) in contact with the branch portions" means the border between the tapered portion, where the diameter change occurs, and the branch portions (B), where the branching begins; the "meeting point (3) of the two branch portions" means a point, on the "line in contact with the branch portions", where splitting into the two branch portions occurs; and the "foot (4) of the line extending, from the meeting point (3) of the two branch portions on the line (2) in contact with the two branch portions, toward, and perpendicularly crossing with, the line (1) in contact with the large-diameter portion" means the point on the "line (1) in contact with the large-diameter portion" where, when a line perpendicular to the "line (1) in contact with the large-diameter portion" is drawn from the "meeting point (3) of the two branch portions", the drawn line perpendicularly crosses with the "line (1) in contact with the large-diameter portion".

As illustrated in FIG. 1, when the sum of the diameters of the two branch portions is smaller than the diameter of the large-diameter portion, the triangular shape corresponding to the portion where a shift in the woven structure occurs has, as the base, a line connecting two arbitrary points on the line (2) in contact with the two branch portions, and has, as the apex, the foot (4) of the line extending from the splitting point (meeting point (3)) of the two branch portions toward, and perpendicularly crossing with, the line (1) in contact with the large-diameter portion. By using the splitting point (meeting point (3)) of the branch portions as the basis, the warp yarns to be used for the left and right branch portions can be appropriately distributed to enable preparation of a wrinkle-free graft.

Alternatively, as illustrated in FIG. 2, when the sum of the diameters of the two branch portions is larger than the diameter of the large-diameter portion, the triangular shape corresponding to the portion where a shift in the woven structure occurs has, as the base, a line connecting two arbitrary points on the line (1) in contact with the large-diameter portion, and has, as the apex, the splitting point (meeting point (3)) of the two branch portions. By using the splitting point (meeting point (3)) of the branch portions as the basis, the warp yarns to be used for the left and right branch portions can be appropriately distributed to enable preparation of a wrinkle-free graft.

The 1/1 plain woven structure is preferably used for the portion(s) with a larger area. When the difference in the area is not large between the large-diameter portion and the branch portions, i.e., when the ratio between the sum of the diameters of the two branch portions and the diameter of the large-diameter portion (the sum of the diameters of the two branch portions / the diameter of the large-diameter portion) is close to 1, the 1/1 plain woven structure may be used for the portion(s) with a larger area, and the 1/1 plain-1/2 rib one-yarn-alternating or two-yarn-alternating woven structure may be used for the portion(s) with a smaller area. When ratio is far from 1, the 1/1 plain woven structure may be used for the portion(s) with a larger area, and the 1/2 rib-alone woven structure may be used for the portion(s) with a smaller area. However, the woven structures used may be appropriately selected and combined according to the shrinkage properties and the surface conditions of the yarns.

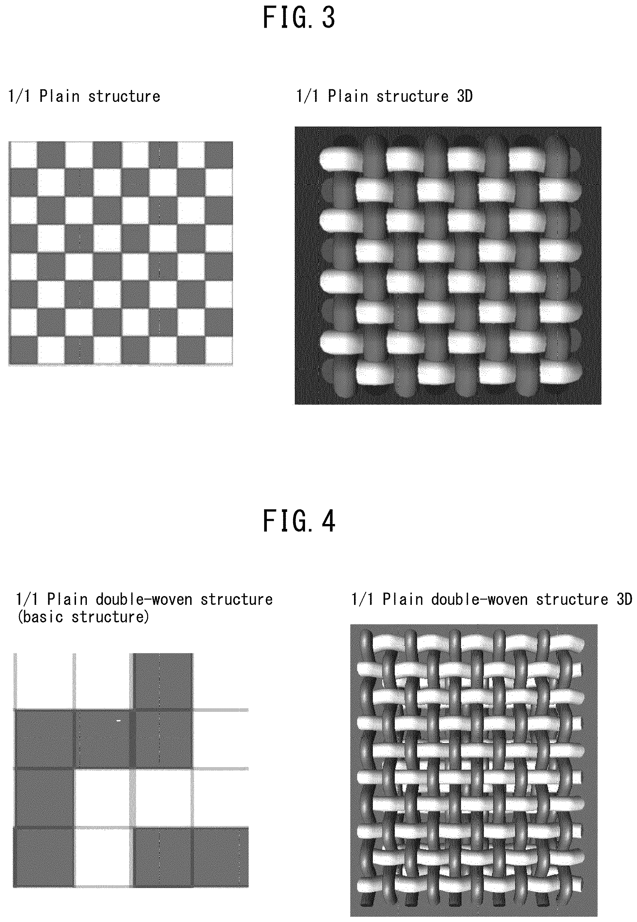

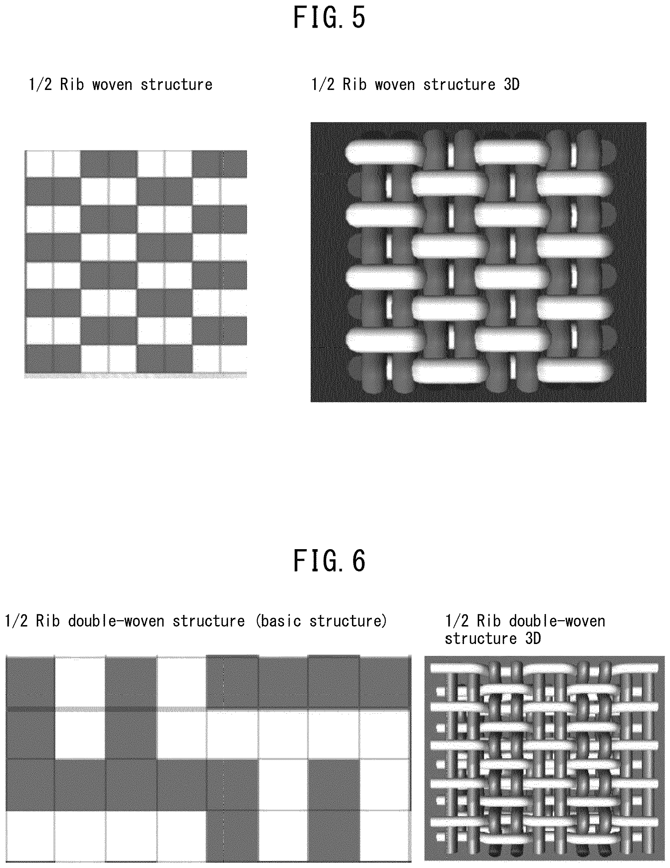

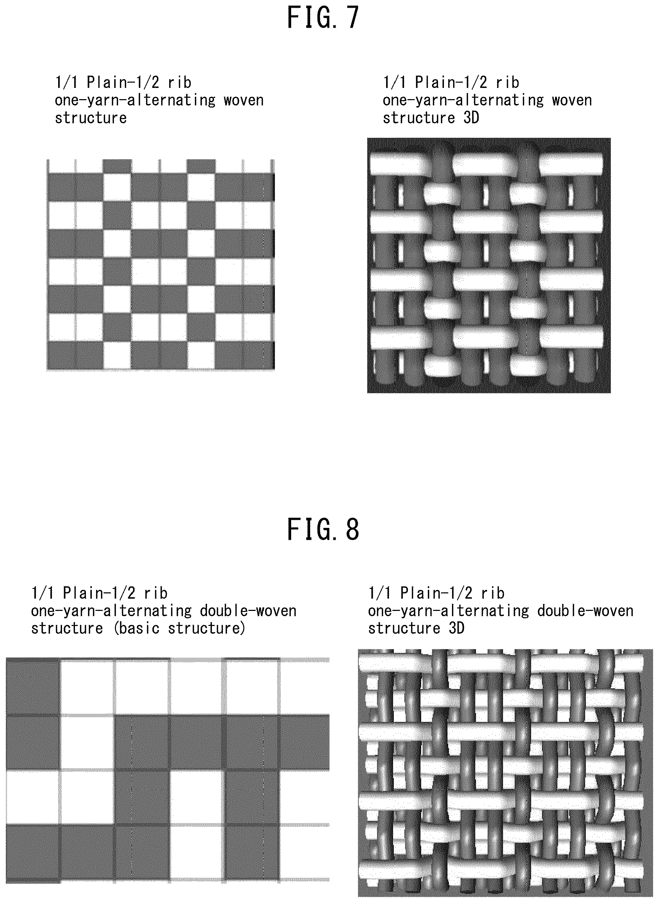

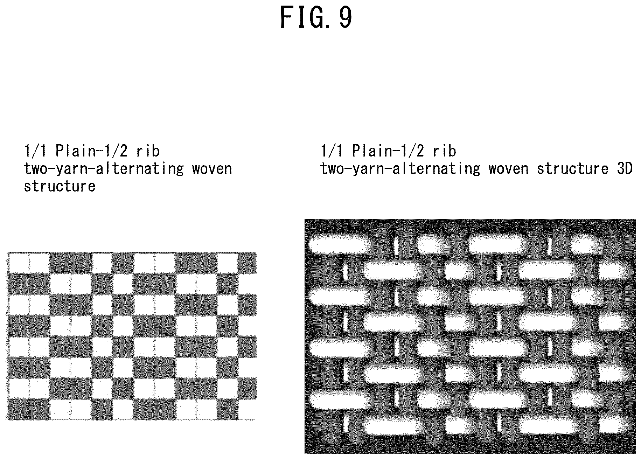

Each woven texture represents the mode of interlacing of warp yarns and weft yarns. As illustrated in FIG. 3, 1/1 plain represents a woven structure in which warp yarns and weft yarns are alternately interlaced up and down. As illustrated in FIG. 5, 1/2 rib is a woven structure in which warp yarns are running up and down (relative to weft yarns) in the one-yarn-alternating manner while the weft yarns are running up and down (relative to warp yarns) in the two-yarn-alternating manner. The 1/1 plain-1/2 rib one-yarn-alternating woven structure is a woven texture such as the one illustrated in FIG. 7. The 1/1 plain-1/2 rib two-yarn-alternating woven texture is a woven structure such as the one illustrated in FIG. 9.

As illustrated in FIGs. 1 and 2, the warp yarn density (the number of warp yarns/2.54 cm) in 1/2 rib is higher than the warp density in 1/1 plain. Therefore, as the ratio of 1/2 rib increases, the diameter decreases when the large-diameter portion and the branch portions are woven using the same number of warp yarns. However, it was found that a direct shift of the woven structure from 1/1 plain to 1/2 rib may cause a rapid change in the diameter, leading to generation of wrinkles in the tapered portion. In view of this, in the present embodiment, as illustrated in FIG. 1, when the sum of the diameters of the two branch portions is smaller than the diameter of the large-diameter portion, the large-diameter portion has a 1/1 plain woven structure; the two branch portions have a 1/2 rib woven structure, or have a 1/1 plain-1/2 rib one-yarn-alternating woven structure; the tapered portion is bounded, in one front view, by a line (1) in contact with the large-diameter portion, and a line (2) in contact with the two branch portions; and shifting from the woven structure of the large-diameter portion to the woven structure of the two branch portions occurs to form a triangular shape from the foot (4) of the line extending, from the meeting point (3) of the two branch portions on the line (2) in contact with the two branch portions, toward, and perpendicularly crossing with, the line (1) in contact with the large-diameter portion, toward two arbitrary points on the line (2) in contact with the two branch portions; so as to conform to the diameter change, to thereby provide woven structures with which generation of wrinkles in the tapered portion can be prevented.

The start point of the shift in the woven structure may be shifted toward the branch-portion side as long as the water permeability and the burst strength are not affected even after modification of the taper under the heat setting conditions. However, a large shift is not preferred, and a shift of up to about 5 mm is appropriate. With the thus constituted woven structure, a seamless tubular woven fabric can be prepared.

In 1/2 rib, two warp yarns constituting 1/1 plain are combined to form one warp yarn having twice the fineness, and the rib structure is generally used for the portion(s) with a smaller diameter. The number of warp yarns used itself does not increase or decrease from the body portion to the branch portions. The number increases or decreases depending on the combination in terms of the woven structures.

The lines bounding the "triangular shape" in the tapered portion may be linear. As presented in the enlarged photographs illustrating the borders in FIGs. 1 and 2, the term "linear" means a stepwise shift of the warp yarns and the weft yarns between woven structures. The shift may occur on every-other-yarn basis, or on every-two- or every-three-yarn basis. The shift in the woven structure may be based on a combination determined taking into account the shrinkage properties of the yarns according to the difference in the woven structure.

In the present description, the term "one front view" means a drawing from any direction perpendicular to the longitudinal direction of the graft. In each of FIGs. 1 and 2, the triangular area in the tapered portion is illustrated in one front view of the tubular woven fabric. In three dimensions, such a triangular area may be provided in a plurality of locations on the tubular circumference. Taking into account the symmetry of the branched graft, the areas are preferably present in an even number of locations. The shape of the triangle in the "triangular shape" is a shape represented by, for example, an isosceles triangle, wherein the woven texture changes bilaterally symmetrically, and linearly, from the apex of the triangular shape. Regarding the size of the shape, a combination of large and/or small triangles may be formed as a whole in the woven fabric. The shape of the triangle may also be a shape in which the apex is not located on the perpendicular bisector of the base. The simplest structure is a structure in which, as illustrated in FIGs. 1 and 2, one triangle is present in front, and the same triangle is present also in the back side thereof.

The woven fabric of the present embodiment needs to have a cover factor of 1600 to 2400 in each of the large-diameter portion and the two branch portions. When the cover factor is not less than 1600, the woven fabric has an appropriate weaving density, and tends not to cause blood leakage from the woven fabric itself. When the cover factor is not more than 2400, the woven fabric has a high weaving density, but reduction of the diameter can still be achieved, and flexibility of the woven fabric itself is also within an acceptable range. The cover factor is preferably 1800 to 2300, more preferably 2000 to 2200. The cover factor in the warp direction and the cover factor in the weft direction are preferably, but do not necessarily need to be, almost the same. When the cover factor in the warp direction is higher, the high density woven fabric can be more easily produced. From the viewpoint of the water permeability and the strength, not only each of the large-diameter portion and the two branch portions, but also the tapered portion preferably has a cover factor of 1600 to 2400. In 1/2 rib, two warp yarns constituting 1/1 plain are combined to form one warp yarn having twice the fineness. Therefore, in the calculation of the cover factor, the yarn number is regarded as 1 while the fineness is doubled.

The cover factor (CF) can be calculated according to the following equation:

$$CF = (\sqrt{dw}) \times Mw + (\sqrt{df}) \times Mf$$

{wherein dw represents the total fineness (dtex) of a warp yarn pulled out from the woven fabric; Mw represents the weaving density (yarns/2.54 cm) of warp yarns; df represents the total fineness (dtex) of a weft yarn pulled out from the woven fabric; and Mf represents the weaving density (yarns/2.54 cm) of weft yarns}.

In the present embodiment, as described above, when the sum of the diameters of the two branch portions is smaller than the diameter of the large-diameter portion, the large-diameter portion has a 1/1 plain woven structure; the two branch portions have a 1/1 plain-1/2 rib one-yarn-alternating or two-yarn-alternating woven structure; and, in the tapered portion, in one front view, shifting from the woven structure of the large-diameter portion to the woven structure of the branch portions linearly occurs to form a triangular shape from a certain point on the line in contact with the large-diameter portion toward the line in contact with the two branch portions, so as to conform to the diameter change and to suppress changes in the warp-yarn cover factor among the large-diameter portion, the two branch portions, and the tapered portion.

Further as described later, in weaving of the woven fabric of the present embodiment, reed beating may be performed using a reed in which the spaces between the dents vary in the vertical direction, to thereby change the diameter of the tubular shape in the longitudinal direction, and/or to control the cover factor.

The woven fabric of the present embodiment is a seamless tubular woven fabric. For a graft to be used for a stent graft, a sheet-like woven fabric or membrane may be formed into a tubular shape, and then the ends may be bonded together using an adhesive, or may be sewn together. However, this increases the thickness in the bonded or sewn portion, to prevent compact folding. Thus, a seamless woven fabric is preferred for the reduction of the diameter. Further, because the weft yarn continuously constitutes the woven fabric, bonding and sewing, which are carried out when a flat, non-tubular woven fabric or membrane material is used, and which are laborious manual processes that cause variations, can be eliminated, and moreover, leakage can be reduced. Further, because of the elimination of the surface roughness, smooth flow of blood can be effectively achieved.

The basic woven structure of the main part of the woven fabric of the present embodiment is the plain weave from the viewpoint of thinness and strength of the woven fabric, and reduction of blood leakage. However, as described above, in the tubular woven fabric of the present embodiment,

when the sum of the diameters of the two branch portions is smaller than the diameter of the large-diameter portion as illustrated in FIG. 1, the large-diameter portion has a 1/1 plain woven structure; the two branch portions have a 1/1 plain-1/2 rib one-yarn-alternating or two-yarn-alternating woven structure; and, in the tapered portion, in one front view, shifting from the woven structure of the large-diameter portion to the woven structure of the branch portions linearly occurs to form a triangular shape

from the foot (4) of the line extending, from the splitting point (meeting point (3)) of the two branch portions toward, and perpendicularly crossing with, the line (1) in contact with the large-diameter portion, toward both ends on the line (2) in contact with the two branch portions; and

when the sum of the diameters of the two branch portions is larger than the diameter of the large-diameter portion as illustrated in FIG. 2, the large-diameter portion has a 1/1 plain-1/2 rib one-yarn-alternating or two-yarn-alternating woven structure; the two branch portions have a 1/1 plain woven structure; and, in the tapered portion, in one front view, shifting from the woven structure of the two branch portions to the woven structure of the large-diameter portion linearly occurs to form a triangular shape

from the splitting point (meeting point (3)) of the two branch portions

toward both ends on the line (1) in contact with the large-diameter portion.

For any of the woven structures described above, each of the warp density and the weft density of the woven fabric of the present embodiment is preferably not less than 100 yarns/2.54 cm, more preferably not less than 120 yarns/2.54 cm, still more preferably not less than 160 yarns/2.54 cm. Although there is no upper limit, the density is substantially not more than 280 yarns/2.54 cm from the viewpoint of weaving.

The woven fabric of the present embodiment needs to have a thickness of not more than 110 $\mu$m in each of the large-diameter portion and the two branch portions. When the woven fabric has a thickness of not more than 110 $\mu$m, reduction of the diameter upon folding can be achieved, and therefore the woven fabric can be contained in a desired catheter. The thickness is preferably within the range of 10 $\mu$m to 100 $\mu$m. In this case, the woven fabric can be easily contained in a catheter having a small diameter, and a delivery system can be provided such that the woven fabric can be easily released upon the release in the affected area. When the woven fabric has a thickness of not less than 10 $\mu$m, a sufficient burst strength can be retained. The thickness of the woven fabric herein is defined as follows. In an area in the circumferential direction and the longitudinal direction (5 cm to 30 cm) of the tubular woven fabric, 10 sites are arbitrary selected, and the thickness at each site is measured using a thickness gauge. The average of the measured values is the thickness of the woven fabric.

For example, the thickest blood vessel for which a stent graft is used is the thoracic aorta, which usually has an inner diameter of about 40 to 50 mm. For reduction of the physical burden on the patient, and for application to a wider range of patients, a stent graft with a maximum inner diameter of 50 mm is required to be insertable into an 18-French (6-mm inner diameter) or smaller catheter in the cases of the thoracic aorta. According to the past studies by the present inventors, it became clear, for example, that the maximum thickness of a tubular woven fabric having an inner diameter of 50 mm that can pass through a hole having a diameter of 6 mm is 90 $\mu$m, and that the maximum thickness of a tubular woven fabric having an inner diameter of 40 mm that can pass therethrough is 110 $\mu$m. Since these thicknesses do not largely change even when the tubular woven fabric has a different inner diameter, the standard for the thickness of the woven fabric is set to 110 $\mu$m or less in the specification of the monofilament fineness and the total fineness of the superfine polyester fiber used in the woven fabric for the stent graft.

The woven fabric of the present embodiment needs to have a burst strength of not less than 100 N as measured by a burst strength test in accordance with ANSI/AAMI/ISO7198 8.3.3: 1998/2001 in each of the large-diameter portion, the two branch portions, and the tapered portion. When the woven fabric has a burst strength of not less than 100 N, when the woven fabric is used as a woven fabric for a stent graft, safety during the use can be secured since, for example, it withstands the expansive force of the stent. The burst strength is preferably not less than 125 N, more preferably not less than 150 N, still more preferably not less than 200 N. There is no upper limit of the burst strength of the woven fabric. From the viewpoint of the balance with the thinness of the woven fabric, the burst strength is substantially not more than 500 N.

The woven fabric itself of the present embodiment preferably has a water permeability of not more than 500 ml/cm$^2$/min in each of the large-diameter portion, the two branch portions, and the tapered portion. The water permeability of the woven fabric is an index for prevention of blood leakage, and, when the water permeability is not more than 500 ml/cm$^2$/min, blood leakage from the wall surface of the woven fabric can be kept low. The water permeability of the woven fabric is more preferably not more than 300 ml/cm$^2$/min, especially preferably 200 ml/cm$^2$/min. There is no lower limit of the water permeability of the woven fabric. It is not less than 0 ml/cm$^2$/min. Usually, by using the woven fabric of the present embodiment as a graft, and sewing the graft to a metal stent using a suture thread, a stent graft is prepared as the final product. When a large needle hole is opened in the woven fabric during this process, blood leakage occurs therefrom. In such cases, the medical woven fabric of the present embodiment preferably has a water permeability of not more than 500 ml/cm$^2$/min after the needle puncture. The water permeability after the needle puncture herein is a value measured after arbitrarily passing a sewing-machine needle (DB×1 normal needle #11, manufactured by Organ Needle Co., Ltd.) 10 times per 1 cm$^2$. For reduction of the size of the needle hole, use of a superfine polyester fiber is effective. This is because, after monofilaments in the woven texture are forced to open by the needle, recovery of the monofilaments occurs due to flexibility of the monofilaments, hardly leaving a needle hole, so that the water permeability after the needle puncture can be kept low.

The tubular woven fabric of the present embodiment includes a large-diameter portion; two branch portions having diameters whose sum is larger or smaller than the diameter of the large-diameter portion; and a tapered portion arranged between the large-diameter portion and the two branch portions. The branched portion is a portion in which the tubular large-diameter portion is continuously branched into two or more branch portions via the tapered portion. Part of the woven texture in the boundary portion between the tapered portion and the branch portions may be a single texture. The single texture may be a structure in which the woven fabric in the upper side and the woven fabric in the lower side are bound to each other. For example, a 2/2 basket structure, 2/2 twill structure, 3/3 basket structure, 3/3 woven structure, or the like may be used as a reasonable structure for the woven structure. The structure may also be a woven structure such as 1/2 rib, 2/1 rib, or 1/1 plain. These may be selected as long as no problem occurs in terms of weaving or handling.

When the woven fabric of the present embodiment has branch portions, the branch portions may have different diameters, and/or may be three or more branches. Although the branch portions may have the same length, one branch is generally longer than the other. This is because, for example, in treatment of an abdominal aneurysm, a catheter containing a stent graft having a long branch portion compressively inserted therein is introduced through the iliac artery in one side so that the stent graft is placed in the aneurysm, and then a short straight stent graft is inserted from the other iliac artery to bind it to the above stent graft. Regarding a stent graft branching from the abdominal aorta to the left and right iliac arteries, the stent graft may have a maximum inner diameter of, for example, 20 to 40 mm in terms of the diameter of the main body, and 10 to 20 mm in terms of the diameter of the branch portions.

When the woven fabric of the present embodiment has branch portions, for example, during weaving of one branch portion, the warp yarns constituting the other branch portion may be on standby at the upper shed position, or may be on standby at the lower shed position. The woven structure may be produced in an easy-to-weave pattern. There are no particular limitations when the number of warp yarns is small and the load on Jacquard machines or dobby machines is small, such as in the case of a woven fabric for a graft. In the case of weaving of a woven fabric including a branched portion, the number of shuttles to be provided is preferably the sum of the number of the branch portions and the number of the large-diameter portion. For example, when two branch portions are woven, three shuttles containing weft yarns are preferably provided. However, since one of the branch portions can be woven with the shuttle used for weaving the large-diameter portion, the weaving is also possible with two shuttles. In the case of a straight fabric having no branch portion, weaving is possible by providing one shuttle containing the weft yarn, and the weft yarn can be continuous. By using two or more shuttles for the weaving, the time interval of replacement of the weft yarn can be increased.

The woven fabric of the present embodiment may be coated with collagen, gelatin, or the like as long as the requirements of the thickness, outer diameter, and the like described above are satisfied.

The woven fabric of the present embodiment is usually used as a stent graft by combination with a stent (spring-shaped metal), which acts as an expandable member. Examples of the type of the stent graft include: a simple straight type, which has a tubular shape; and a branched type and a fenestrated type, which are applicable to branched blood vessels. For the expandable member, a self-expanding material using a shape-memory alloy, superelastic metal, or synthetic polymer material may be used. The expandable member may have any of the designs of the conventional techniques. For example, the woven fabric of the present embodiment may be used as a graft, and a zigzag metal stent may be sutured and fixed to the inner face and/or outer face of the woven fabric using a suture thread. As the expandable member, a member that is expanded by a balloon may be applied instead of the self-expanding material. In a stent graft as a preferred mode of the present invention, the gap between the stent and the graft is preferably not more than 2 mm.

Both the warp yarns and the weft yarns used in the present embodiment are preferably polyester fibers. In particular, the superfine polyester fiber constituting the weft yarn preferably has a tensile strength of not less than 3.5 cN/dtex and a tensile elongation of not less than 12%. When the tensile strength of the superfine polyester fiber is not less than 3.5 cN/dtex, the woven fabric for a stent graft can produce excellent mechanical/physical properties. Polyester fibers can have an increased tensile strength by increasing the draw ratio, and further, even when the tensile strength is increased to, for example, not less than 3.5 cN/dtex by drawing, the tensile elongation of not less than 12% imparts toughness, leading to prevention of rupture or breakage due to impact. From the viewpoint of stable processability in the weaving process of the woven fabric, the superfine polyester fiber in the present embodiment more preferably has a tensile strength of not less than 3.8 cN/dtex, still more preferably not less than 4.0 cN/dtex. From a similar viewpoint, the superfine polyester fiber in the present embodiment more preferably has a tensile elongation of not less than 15%, still more preferably not less than 20%. Since the superfine polyester fiber has a low monofilament fineness, it tends to generate fluff. Thus, a coating may be formed on the yarn by application of a sizing agent, lubricant, or WAX agent, and/or ease of handling during the weaving may be improved by improving the bundling property of the yarn by twisting or the like.

In weaving of the woven fabric of the present embodiment, the warp yarn may be subjected to twisting at 50 to 1000 T/m, and the twisted yarn may be further subjected to application of a sizing agent, lubricant, or WAX agent. Even without the twisting, application of a sizing agent, lubricant, or WAX agent is effective for suppressing the fluff during the weaving, to improve the weaving performance. However, from the viewpoint of biological safety, sizing is preferably not carried out, and twisting at 300 to 700 T/m alone is preferably carried out for warping of the warp yarn. Even in this case, the spinning oil agent during the production of the original yarn is adhering to the warp yarn. The weft yarn may also be subjected to, for example, further application of a spinning oil agent or another oil agent, and/or may be subjected to, for example, twisting at about 50 to 200 T/m, to decrease friction and hence to improve the weaving performance. A method suitable for the weaving may be employed as appropriate.

Examples of materials constituting the woven fabric of the present embodiment, other than the superfine polyester fiber, include polyester fibers other than those described above, polyamide fibers, polyethylene fibers, and polypropylene fibers. These may be either monofilaments or multifilaments, and may be used in combination with one or more fiber materials depending on the purpose. Regarding the mode of the combination, a polyester fiber described above may be twisted with another fiber to provide a composite fiber, or another fiber may be used as, or may be partially used as a part of, the warp yarn or the weft yarn of the woven fabric.

In the superfine polyester fiber, the content of the PET component is preferably not less than 98% by weight, i.e., the content of the components other than PET is preferably less than 2% by weight. The "components other than PET" herein means components incorporated into the molecular chains by copolymerization or the like, and components adhering to the surface of the polyester fiber, such as copolymerized PET, polyamide and polystyrene, and copolymers thereof, sea component polymers used for production of sea-island superfine PET fibers, such as polyethylene and polyvinyl alcohol, and degradation products of the sea component polymers. In the present embodiment, the components other than PET preferably do not include PET-derived monomers or oligomers such as ethylene glycol, terephthalic acid (TPA), monohydroxyethylene terephthalate (MHET), or bis-2-hydroxyethyl terephthalate (BHET). The content of the components other than PET in the superfine polyester fiber is preferably less than 1% by weight, more preferably less than 0.5% by weight, still more preferably 0.

In the woven fabric of the present embodiment, polyester fibers, especially superfine polyester fibers, effectively function not only for a woven fabric for a stent graft, but also as constituent fibers of a material to be implanted in the body, such as an artificial blood vessel, artificial fiber cloth, antiadhesive agent, or artificial valve. Further, the fibers effectively function not only for the material to be implanted in the body, but also as constituent fibers for a medical material to be used outside the body, such as a hemofiltration material, cell separation membrane, cell adsorbent, or cell culture substrate. The polyester fibers, especially the superfine polyester fibers, can be used, of course, not only in the medical field, but also as a material for cloths or as a material such as a filter or a wiping material.

The woven fabric of the present embodiment is preferably a woven fabric composed of warp yarns and weft yarns from the viewpoint of achievement of the strength of the stent graft and prevention of blood leakage. From the viewpoint of thinness of the woven fabric, the woven fabric of the present embodiment needs to contain not less than 20% by weight superfine polyester fibers. When the component ratio of the superfine polyester fibers in the present embodiment in the woven fabric is not less than 20% by weight, the woven fabric can easily achieve a thickness of not more than 110 μm, so that reduction of the diameter can be easily realized. Further, when the component ratio of the superfine polyester fibers is not less than 20% by weight, excellent integration with the stent can be achieved. In the woven fabric of the present embodiment, the component ratio of the superfine polyester fibers is preferably not less than 30% by weight, more preferably not less than 40% by weight. Although the superfine polyester fibers in the present embodiment may be used for both the warp yarn and the weft yarn of the woven fabric, the superfine polyester fibers are especially preferably used for the weft yarn from the viewpoint of better integration with the stent.

In the method of producing a superfine polyester fiber suitable for use in the woven fabric of the present embodiment, a finishing agent may be applied to a fiber bundle to improve processability during the subsequent warping and weaving processes. As the finishing agent, a mineral oil-derived lubricant, a water-soluble lubricant, or the like is used. The oil application rate of the finishing agent is preferably 1% by weight to 3% by weight, more preferably 1.2% by weight to 2.8% by weight, still more preferably 1.5% by weight to 2.5% by weight, from the viewpoint of processability during the bulking process and the weaving/knitting process.

In the method for producing the superfine polyester fiber, tangling treatment is preferably carried out at an undrawn-yarn stage or a drawn-yarn stage from the viewpoint of reducing fluff and yarn breakage during the warping and knitting/weaving processes, and improving the unwinding property. For the tangling treatment, a known tangling nozzle is preferably employed, and the number of tangles is preferably within the range of 1 to 50 tangles/m. The thermal shrinkage stress of the superfine polyester fiber used in the weaving is preferably not less than 0.2 cN/dtex within the temperature range of 80°C to 200°C, from the viewpoint of securing a thermal shrinkage stress of not less than 0.05 cN/dtex as a superfine polyester fiber constituting the woven fabric of the final product of the stent graft (after sterilization treatment).

In a preferred mode of the present embodiment, the stent graft is delivered through blood vessels in a state where the stent graft is inserted in a catheter. Since the stent graft in the present embodiment uses a woven fabric having a thickness of not more than 110 μm, it is thin and highly flexible. It can therefore be inserted into a small-diameter catheter, and, as a result, the stent graft can be easily delivered through blood vessels with a reduced risk of damaging the vascular wall. As the catheter, those using conventional techniques, such as a tube-type catheter or balloon-type catheter, may be preferably used. The stent graft inserted in a small-diameter catheter in the present embodiment can be delivered through blood vessels and placed therein using a conventional delivery system. When the tubular seamless woven fabric of the present embodiment is used as a woven fabric for a stent graft, the diameter of the stent graft can be reduced, so that the physical and economic burdens on the patient can be reduced by, for example, shortening of the hospitalization period. Further, risks such as damaging the vascular wall can be reduced. Further, transcatheter endovascular treatment can be applied to a wider range of cases to which the treatment has not been applicable so far, including cases of females and Asians having thin arteries.

The production of the woven fabric of the present embodiment is described below. In the step of providing the warp yarn constituting the woven fabric of the present embodiment, a required number of warp yarns are wound up on a warp beam using a warping machine, and the warp beam may be loaded in a loom. Alternatively, the warp yarns loaded in a creel may be directly drawn onto a loom.

The loom used for the production of the seamless tubular woven fabric of the present embodiment is not limited. It is preferred to use a shuttle loom in which the weft yarn is passed through by reciprocal movement of a shuttle, for production of the seamless woven fabric, and also for suppressing variation of the weaving density of the selvage portion of the woven fabric (the folded portion of the tubular woven fabric) to make the thickness of the woven fabric uniform. When a shuttle loom is used, when there are two branch portions, the weaving may be carried out using three shuttles for the large-diameter portion, one branch portion, and the other branch portion, respectively. Alternatively, when two shuttles are used, the weaving may be carried out using one shuttle for the large-diameter portion and one branch portion, and using the other shuttle for the other branch portion. By application of a constant tension during unwinding of the weft yarn from each shuttle, a high-quality tubular woven fabric having no wrinkles can be effectively woven. The drawing tension for the weft yarn may be made uniform with, for example, a structure using a plurality of springs, or a compact motor. As described above, when the woven fabric of the present embodiment is a straight fabric having no branched portion, weaving is possible by providing at least one shuttle containing the weft yarn, and the weft yarn can be continuous.

In weaving of a tubular woven fabric such as the woven fabric in the present embodiment, a full-width temple may be used for the purpose of stabilizing the cloth fell, attaining a uniform thickness and diameter of the woven fabric, and suppressing yarn breakage and the like during processing. For the member of the full width temple in the portion in contact with the woven fabric, a material having a low friction coefficient is preferably selected. For the surface of the take-up roll, a tacky, non-slippery material having a smooth surface is preferably used. Regarding the structure of the full-width temple and the frictional coefficients of the members used, an appropriate design may be selected according to the monofilament fineness and the total fineness of each yarn used, and the weaving densities of the warp yarn and the weft yarn.

The weaving of the tubular seamless woven fabric requires a control of raising and lowering of the warp yarn. As the apparatus therefor, a Jacquard shedding apparatus, a dobby shedding apparatus, or the like may be employed. For easier formation of the woven texture of the branched portion, an electronic Jacquard machine is especially preferably used.

For changing the diameter of the tubular shape in the longitudinal direction, and/or for controlling the cover factor, as described above, the woven fabric may be prepared by performing reed beating using a reed in which the spaces between the dents vary in the vertical direction to thereby vertically change the reed beating position, or performing reed beating while vertically moving the weaving end.

After the weaving, in post-processing, scouring treatment for the purpose of removing the lubricant and the like, and heat setting for the purpose of shape stabilization are carried out. The temperature and the treatment time for the scouring, the temperature and the treatment time for the heat setting, and the tension in each process are not limited. Regarding the post-processing conditions, for example, the woven fabric may be treated under the following conditions: pre-heat setting at 150°C for 30 minutes, scouring at 90°C for 30 minutes, drying at 60°C for 30 minutes, and final heat setting at 185°C for 10 minutes. The treatment conditions may be appropriately determined according to the properties of the woven fabric.

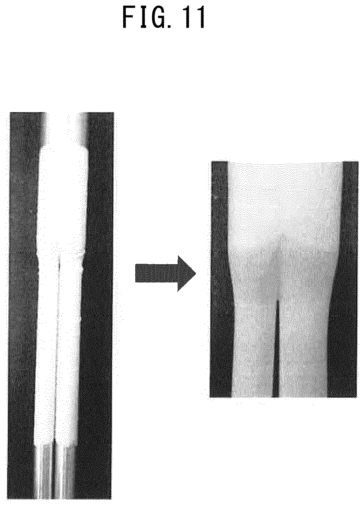

As illustrated in FIG. 11, when the woven fabric of the present embodiment is subjected to post-processing, a metal jig for heat setting (heat setting bar) is preferably prepared as follows. A stainless steel tube having the diameter of the large-diameter portion is connected to tapered stainless steel tubes having the diameter of the branch portions such that no boundary appears. When there is a single texture in the vicinity of the branch portions, the diameter of the jig is reduced correspondingly to the reduction of the diameter due to the single texture. Preferably, from the viewpoint of workability, metal jigs for the large-diameter portion and the branch portions are separately prepared such that they have structures enabling insertion of the metal jigs from the top and the bottom into the woven fabric to be subjected to the heat setting, and enabling their fixation in the woven fabric, or metal jigs for the large-diameter portion and the branch portions separately prepared may be assembled to provide a structure enabling insertion of the woven fabric to the resulting metal jig from the branch-portion side, to fix the woven fabric having the desired diameters without wrinkles. By performing such post-processing, a wrinkle-free woven fabric for a branched graft can be produced.

The treated woven fabric is combined with a stent using a suture thread. The conditions for the joining of the woven fabric with the stent may be selected according to the shape of the stent. The needle used for the suture is not limited, and is preferably selected such that the water permeability after the needle puncture is not more than 500 ml/cm$^2$/min. Subsequently, the stent graft obtained by the above method is subjected to sterilization treatment. The conditions for the sterilization treatment are not limited, and may be selected taking into account the balance between the sterilization effect and the thermal shrinkage stress of the superfine polyester fiber after the treatment.

EXAMPLES

The present invention is concretely described below by way of Examples. However, the present invention is not limited to the Examples. Common measurement values for physical properties were measured by the following methods.

(1) Total Fineness and Monofilament Fineness

The total fineness (dtex) is measured for a 10-cm fiber bundle cut out from the large-diameter portion of the woven fabric. In the case of the warp yarn, the large-diameter portion is cut in the warp direction, and a warp yarn is pulled out from the cut end. In the case of the weft yarn, a spirally textured weft yarn is pulled out. The pulled-out yarn was dried to absolute dryness for 1 hour in an oven at 110°C. The yarn was then subjected to measurement of the weight using an analytical balance (SHIMADZU/AUW320), and the weight (g) was read to four decimal places, followed by calculation of the fineness (fineness based on corrected weight, F0) according to the following equation:

$$F0 = 1000 \times (m/L) \times \{(100 + R0)/100\}$$

{wherein F0 represents the fineness based on corrected weight (dtex); L represents the length of the sample (m); m represents the absolute dry mass of the sample; and R0 represents the official regain (%) defined in 3.1 of JIS-L-0105}.
The measurement was carried out 10 times for each case, and the average was rounded to the nearest integer.
The monofilament fineness (dtex) is the value obtained by dividing the total fineness calculated by the above method, by the number of monofilaments.
The total fineness of the branch portions can be measured in the same manner as the large-diameter portion.
When the 10-cm fiber bundle cannot be sampled from the large-diameter portion or the branch portions, the longest fiber bundle that can be sampled from an area not overlapping with the tapered portion may be used to measure the total fineness by the same method.

(2) Weaving Density

A square piece of at least 20 mm × 20 mm was cut out from the woven fabric, and placed on a flat table. After removal of wrinkles, a pick counter (TEXTEST/FX3250) was perpendicularly placed with respect to the warp direction, and the warp density was measured. The displayed integer value was read. The measurement was carried out five times at different sites in the longitudinal direction of the woven fabric, and the average was rounded to one decimal place.
The weft density was measured in the same manner.

(3) Cover Factor

Based on the total fineness determined in (1) and the weaving density determined in (2), the cover factor was calculated according to the following equation:

$$CF = (\sqrt{dw}) \times Mw + (\sqrt{df}) \times Mf$$

{wherein dw represents the total fineness (dtex) of the warp yarn pulled out from the woven fabric; Mw represents the weaving density (yarns/2.54 cm) of the warp yarn; df represents the total fineness (dtex) of the weft yarn pulled out from the woven fabric; and Mf represents the weaving density (yarns/2.54 cm) of the weft yarn}.
The CF was rounded to the nearest integer. In the calculation of the cover factor for a rib texture, since two warp yarns constituting the plain woven texture are combined to form one warp yarn having twice the fineness, the number of warp yarn was regarded as 1 while the fineness was doubled.

(4) Twist Number

**[0075]** The twist number was measured for 10 yarns having a length of 100 mm pulled out from the large-diameter portion of the taper-shaped graft. The measurement was carried out for each of the warp yarn and the weft yarn.

When the 100-mm fiber bundle cannot be sampled from the large-diameter portion, the longest fiber bundle that can be sampled from an area not overlapping with the tapered portion may be used to measure the total fineness by the same method.

(5) Tensile Strength and Tensile Elongation

Regarding the tensile strength and the tensile elongation, a 300-mm yarn before weaving was collected according to JIS-L-1013, and measurement was carried out 10 times for each of the warp yarn and the weft yarn. For the measurement, Tensilon (EZ-LX), manufactured by Shimadzu Access Corporation, was used.

(6) Burst Strength of Woven Fabric

According to ISO-7198, a burst strength test was carried out for the woven fabric. The base fabric was cut out as a piece of 40 mm × 40 mm from each portion (large-diameter portion, tapered portion, or branch portion), and subjected to the measurement. In the sample collection from the tapered portion, the sample size of 40 mm × 40 mm was secured by including the large-diameter portion and the branch portion as the top portion and the bottom portion, respectively, such that they have the same length. For example, in a case where the tapered portion has a length of 20 mm, a 10-mm area in the large-diameter portion and a 10-mm area in the branch portion were included as the top portion and the bottom portion, respectively. The measurement was carried out after placing the sample such that the tapered portion was positioned at the center. When a sample having a sufficient size cannot be collected in the sample collection from the branch portion, the sample to be measured may be collected such that the sample can be set to the jig for the burst strength. When the size was 30 mm × 30 mm or the like, this fact may be recorded.

The measurement was carried out five times, and the average was rounded to the nearest integer.

(7) Water Permeability of Woven Fabric

According to ISO-7198, the water permeability of the woven fabric was measured. The base fabric was cut out as a piece of 20 × 20 mm from each portion (large-diameter portion, tapered portion, or branch portion), and subjected to the measurement. The measurement was carried out five times, and the average was rounded to the nearest integer.

(8) Thickness of Woven Fabric

The base fabric was cut out as a piece of 20 × 20 mm from each portion (large-diameter portion, tapered portion, or branch portion), and the measurement was carried out for arbitrary sites (n=10) using a thickness gauge according to ISO-7198 to read the thickness ($\mu$m). The resulting average was rounded to the nearest integer. FFD-10, manufactured by Ozaki Mfg. Co., Ltd., was used for the measurement.

(9) Wrinkle Conditions of Tapered Portion

The woven fabric prepared was placed on a flat plate without applying any force, and the presence or absence of wrinkles in the tapered portion was evaluated by visual observation by three individuals. The evaluation supported by two or more individuals was employed.

(10) Insertability into Catheter

A woven fabric to which a stent was sutured was folded such that no unevenness occurred in the circumferential direction as seen from directly above, and whether or not it can be inserted into a catheter having a tubular inner diameter of 6 mm was evaluated. When the insertion was easy, a rating of "Easy" was given; when the insertion was hard, a rating of "Possible" was given; and, when the insertion was impossible, a rating of "Impossible" was given. Five samples were prepared for each condition, and evaluated.

[Example 1]

As the warp yarn, a polyester fiber having a total fineness of 46 dtex/24F, a monofilament fineness of 1.9 dtex, a tensile strength of 4.7 cN/dtex, and a tensile elongation of 37%, as measured for a yarn pulled out from the woven fabric, was used. As the weft yarn, a superfine polyester fiber having a total fineness of 26 dtex/140 F, a monofilament fineness of 0.19 dtex, a tensile strength of 4.1 cN/dtex, and a tensile elongation of 60% was used. Using a shuttle loom provided with an electronic Jacquard shedding apparatus together with three shuttles, a branched tubular seamless woven fabric was prepared. In this woven fabric, the diameter of the large-diameter portion was larger than the sum of the diameters of the two branch portions. In the weaving, the number of warp yarns was 642; the reed width for warp yarns was 54.2 mm; the reed density was 14.8 dents/cm; and the number of warp yarns passing through reed dents was 8 yarns/dent.

Regarding the woven textures in the tapered portion, as illustrated in FIG. 1, a shift in the woven texture was made to form, in a front view, a triangular shape

from the foot of the line extending from the meeting point of the two branch portions toward, and perpendicularly crossing with, the line in contact with the large-diameter portion,

toward both ends of the line in contact with the two branch portions;

such that a 1/1 plain-1/2 rib one-yarn-alternating woven structure gradually expands in 1/1 plain. By forming the same textures also in the back side, lines were made to cross at both ends on the line in contact with the branch portions, and the branch-portion side was made to be entirely composed of the 1/1 plain-1/2 rib one-yarn-alternating woven structure.

The woven fabric after the weaving was subjected to pre-heat setting, scouring, and heat setting under the following treatment conditions, to prepare a tubular woven fabric having an inner diameter of 28.0 mm and a length of 173 mm for the large-diameter portion (body), a length of 20 mm for the tapered portion, an inner diameter of 10.5 mm and a length of 153 mm for a branch portion, and an inner diameter of 11.5 mm and a length of 153 mm for a branch portion.

(Pre-Heat Setting Conditions)

• Pre-heat setting is carried out at 150°C for 30 minutes.

(Scouring Conditions)

• With ultrapure water at 90°C, 30 minutes of washing is carried out twice by weak stirring.
• Fixed-length drying is biaxially carried out at 60°C for 30 minutes.

(Final Heat Setting Conditions)

• A stainless-steel bar having the following sizes: 28 mm (diameter) × 180 mm for the large-diameter portion; 10.5 mm (diameter) and 11.5 mm (diameter), and a length of 200 mm for the branch portions; and a taper length of 20 mm; is inserted into the scoured and dried woven fabric, and both ends of the branched tubular seamless woven fabric are set and fixed using hose bands while the woven fabric are lightly stretched by hand without causing wrinkles or looseness.

• The stainless-steel bar to which the woven fabric is immobilized is placed in an incubator at 185°C. From the time point when the temperature in the incubator is controlled at 185°C, heat setting is carried out for 10 minutes.

The properties and the like of each of the large-diameter portion, tapered portion, 10.5-diameter branch portion, and 11.5-diameter branch portion in the branched tubular seamless woven fabric prepared in Example 1 are presented in Table 1. The weft density of the woven fabric was 177.5 yarns/25.4 mm.

[Example 2]

A branched tubular seamless woven fabric was prepared using the same yarns as in Example 1 as the warp yarn and the weft yarn, and using the same apparatus, but using a different weaving method. In this woven fabric, the diameter of the large-diameter portion was smaller than the sum of the diameters of the two branch portions.

Regarding the woven textures in the tapered portion, as illustrated in FIG. 2, a shift in the woven texture was made to form, in a front view, a triangular shape

from the meeting point of the two branch portions
toward both ends of the line in contact with the large-diameter portion;
such that a 1/1 plain-1/2 rib one-yarn-alternating woven structure gradually expands in 1/1 plain. By forming the same textures also in the back side, lines were made to cross at both ends on the line in contact with the large-diameter portion, and the branch-portion side was made to be entirely composed of the 1/1 plain-1/2 rib one-yarn-alternating woven structure.

The pre-heat setting and the final heat setting of the woven fabric after the weaving were carried out by the same methods as in Example 1 using a stainless steel bar having the following sizes: 22.5 mm (diameter) × 180 mm for the large-diameter portion; 13.5 mm (diameter) and 14.5 mm (diameter), and a length of 200 mm for the branch portions; and a taper length of 20 mm. The properties and the like of each of the large-diameter portion, tapered portion, and branch portions in the branched tubular seamless woven fabric prepared are presented in Table 1. The weft density of the woven fabric was 177.5 yarns/25.4 mm.

[Example 3]

A branched tubular seamless woven fabric was prepared using the same yarns as in Example 1 as the warp yarn and the weft yarn, and using the same apparatus and method. In the branched tubular seamless woven fabric, the diameter of the large-diameter portion was larger than the sum of the diameters of the two branch portions.

However, regarding the woven textures in the tapered portion, as illustrated in FIG. 1, a shift in the woven structure was made to form, in a front view, a triangular shape
from the foot of the line extending from the meeting point of the two branch portions toward, and perpendicularly crossing with, the line in contact with the large-diameter portion,
toward both ends of the line in contact with the two branch portions;
such that a 1/1 plain-1/2 rib two-yarn-alternating woven structure gradually expands in 1/1 plain. By forming the same textures also in the back side, lines were made to cross at both ends on the line in contact with the branch portions, and the branch-portion side was made to be entirely composed of the 1/1 plain-1/2 rib two-yarn-alternating woven structure.

The pre-heat setting, scouring, and final heat setting of the woven fabric after the weaving were carried out by the same methods as in Example 1.

The properties and the like of each of the large-diameter portion, tapered portion, and branch portions in the branched tubular seamless woven fabric prepared are presented in Table 1. The weft density of the woven fabric was 177.5 yarns/25.4 mm.

[Example 4]

A branched tubular seamless woven fabric was prepared with the same apparatus and method as in Example 1 except that, while the same warp yarn as in Example 1 was used, the same yarn as this warp yarn was used as the weft yarn to change the total fineness and the weaving density of the weft yarn. In the branched tubular seamless woven fabric, the diameter of the large-diameter portion was larger than the sum of the diameters of the two branch portions.

The woven structures in the tapered portion were the same as those described in Example 1. The treatment conditions for the woven fabric after the weaving were also the same as those in Example 1.

The properties and the like of each of the large-diameter portion, tapered portion, and branch portions in the branched tubular seamless woven fabric prepared are presented in Table 1. The weft density of the woven fabric was 134 yarns/25.4 mm.

[Example 5]

Weaving and treatment were carried out in the same manner as in Example 1 except that the number of warp yarns was 496; the reed width for warp yarns was 55.9 mm; and the number of warp yarns passing through reed dents was 6 yarns/dent. In the branched tubular seamless woven fabric, the diameter of the large-diameter portion was larger than the sum of the diameters of the two branch portions.

The properties and the like of each of the large-diameter portion, tapered portion, and branch portions in the branched tubular seamless woven fabric prepared are presented in Table 1. The weft density of the woven fabric was 143 yarns/25.4 mm.

[Example 6]

Weaving and treatment were carried out in the same manner as in Example 1 except that the number of warp yarns was 704; the reed width for warp yarns was 56.0 mm; the reed density was 15.7 dents/cm; and the number of warp yarns passing through reed dents was 8 yarns/dent. In the branched tubular seamless woven fabric, the diameter of the large-diameter portion was larger than the sum of the diameters of the two branch portions.

The properties and the like of each of the large-diameter portion, tapered portion, and branch portions in the branched tubular seamless woven fabric prepared are presented in Table 1. The weft density of the woven fabric was 184 yarns/25.4 mm.

[Example 7]

A branched tubular seamless woven fabric was prepared using the same yarns as in Example 1 as the warp yarn and the weft yarn, and using the same apparatus and method. In the branched tubular seamless woven fabric, the diameter of the large-diameter portion was larger than the sum of the diameters of the two branch portions.

However, regarding the woven textures in the tapered portion, a shift in the woven texture was made to form, in a front view, a triangular shape

from the foot of the line extending from the meeting point of the two branch portions toward, and perpendicularly crossing with, the line in contact with the large-diameter portion,

toward both ends of the line in contact with the two branch portions;

such that a 1/2 rib woven structure gradually expands. By forming the same structures also in the back side, lines were made to cross at both ends on the line in contact with the branch portions, and the branch-portion side was made to be entirely composed of the 1/2 rib woven structure.

The pre-heat setting, scouring, and final heat setting of the woven fabric after the weaving were carried out by the same methods as in Example 1.

The properties and the like of each of the large-diameter portion, tapered portion, and branch portions in the branched tubular seamless woven fabric prepared are presented in Table 1. The weft density of the woven fabric was 177.5 yarns/25.4 mm.

[Comparative Example 1]

A branched tubular seamless woven fabric was prepared using the same yarns as in Example 1 as the warp yarn and the weft yarn, and using the same apparatus and method.

However, the woven texture for each of the large-diameter portion, tapered portion, and branch portions was 1/1 plain.

The pre-heat setting, scouring, and final heat setting of the woven fabric after the weaving were carried out by the same methods as in Example 1.

The properties and the like of each of the large-diameter portion, tapered portion, and branch portions in the branched tubular seamless woven fabric prepared are presented in Table 1. The weft density of the woven fabric was 150 yarns/25.4 mm.

[Table 1]

| | | | Example 1 | Example 2 | Example 3 | Example 4 | Example 5 | Example 6 | Example 7 | Comparative Example 1 |
|---|---|---|---|---|---|---|---|---|---|---|
| Yarn | Warp yarn | Yarn type | Regular polyester fiber | Regular polyester fiber | Regular polyester fiber | Regular polyester fiber | Regular polyester fiber | Regular polyester fiber | Regular polyester fiber | Regular polyester fiber |
| | | Total fineness (dtex) | 46 | 46 | 46 | 46 | 46 | 46 | 46 | 46 |
| | | Monofilament fineness (dtex) | 1.9 | 1.9 | 1.9 | 1.9 | 1.9 | 1.9 | 1.9 | 1.9 |
| | Weft yarn | Yarn type | Superfine polyester fiber | Superfine polyester fiber | Superfine polyester fiber | Regular polyester fiber | Superfine polyester fiber | Superfine polyester fiber | Superfine polyester fiber | Superfine polyester fiber |
| | | Total fineness (dtex) | 26 | 26 | 26 | 46 | 26 | 26 | 26 | 26 |
| | | Monofilament fineness (dtex) | 0.19 | 0.19 | 0.19 | 1.9 | 0.19 | 0.19 | 0.19 | 0.19 |
| Woven structure | | Large-diameter portion | 1/1 Plain | 1/1 Plain-1/2 rib one-yarn-alternating | 1/1 Plain | 1/1 Plain | 1/1 Plain | 1/1 Plain | 1/1 Plain | 1/1 Plain |
| | | Tapered portion | Combination of "1/1 plain-1/2 rib one-yarn-alternating woven structure" and "1/1 plain woven structure" | Combination of "1/1 plain-1/2 rib one-yarn-alternating woven structure" and "1/1 plain woven structure" | Combination of "1/1 plain-1/2 rib two-yarn-alternating woven structure" and "1/1 plain woven structure" | Combination of "1/1 plain-1/2 rib one-yarn-alternating woven structure" and "1/1 plain woven structure" | Combination of "1/1 plain-1/2 rib one-yarn-alternating woven structure" and "1/1 plain woven structure" | Combination of "1/1 plain-1/2 rib one-yarn-alternating woven structure and "1/1 plain woven structure" | Combination of "1/2 rib woven structure" and "1/1 plain woven structure" | |
| | | Branch portion | 1/1 Plain-1/2 rib one-yarn-alternating | 1/1 Plain | 1/1 Plain-1/2 rib two-yarn-alternating | 1/1 Plain-1/2 rib one-yarn-alternating | 1/1 Plain-1/2 rib one-yarn-alternating | 1/1 Plain-1/2 rib one-yarn-alternating | 1/2 Rib | 1/1 Plain |

| | | | | | | | | | | |
|---|---|---|---|---|---|---|---|---|---|---|
| Woven fabric evaluation | Cover factor | Large-diameter portion | 2127 | 2157 | 2127 | 2131 | 1701 | 2317 | 2127 | 2127 |
| | | Branch portion (L) | 2157 | 2127 | 2157 | 2160 | 1724 | 2350 | 2005 | 2320 |
| | | Branch portion (R) | 2157 | 2127 | 2157 | 2160 | 1724 | 2350 | 2005 | 2320 |
| | Thickness ($\mu$m) | Large-diameter portion | 81 | 88 | 79 | 95 | 70 | 104 | 80 | 81 |
| | | Tapered portion | 99 | 99 | 98 | 110 | 85 | 124 | 97 | 102 |
| | | Branch portion (L) | 86 | 84 | 82 | 102 | 73 | 111 | 87 | 110 |
| | | Branch portion (R) | 85 | 84 | 82 | 101 | 74 | 112 | 86 | 109 |
| | Burst strength (N) | Large-diameter portion | 220 | 231 | 222 | 269 | 205 | 278 | 219 | 221 |
| | | Tapered portion | 229 | 228 | 235 | 255 | 201 | 271 | 224 | 192 |
| | | Branch portion (L) | 232 | 227 | 240 | 246 | 206 | 264 | 224 | 174 |
| | | Branch portion (R) | 230 | 224 | 233 | 243 | 200 | 269 | 229 | 168 |

EP 3 733 122 A1

(continued)

| | | | | | | | | | |
|---|---|---|---|---|---|---|---|---|---|
| Water permeability (ml/cm²/min) | Large-diameter portion | 90 | 110 | 93 | 320 | 450 | 84 | 84 | 97 |
| | Tapered portion | 95 | 97 | 94 | 376 | 487 | 90 | 97 | 548 |
| | Branch portion (L) | 116 | 88 | 121 | 318 | 443 | 85 | 127 | 231 |
| | Branch portion (R) | 106 | 85 | 112 | 289 | 431 | 83 | 125 | 219 |
| Wrinkle condition in tapered portion | | No wrinkles | No wrinkles | No wrinkles | No wrinkles | No wrinkles | No wrinkles | No wrinkles | Presence of wrinkles |
| Insertability into catheter (6-mm hole) | | Easy | Easy | Easy | Easy | Easy | Possible | Easy | Easy |

20

INDUSTRIAL APPLICABILITY

The seamless tubular medical high density woven fabric according to the present invention has low thickness and high strength, and enables reduction of the diameter. Further, when the woven fabric includes: a large-diameter portion; two branch portions having diameters whose sum is larger or smaller than the diameter of the large-diameter portion; and a tapered portion arranged between the large-diameter portion and the two branch portions; the tapered portion can have a particular structure showing a shift in the woven structure so as to conform to the diameter change. Thus, the woven fabric can be suitably used as a graft for a stent graft in which the graft is sutured and fixed to a metal stent using a suture thread.

**Claims**

**1.** A seamless tubular medical high density woven fabric, satisfying the following (1) to (4):

(1) both warp yarn and weft yarn are synthetic multifilament fibers having a total fineness of not more than 60 dtex;
(2) the woven fabric comprises: a large-diameter portion; two branch portions having diameters whose sum is larger or smaller than the diameter of the large-diameter portion; and a tapered portion arranged between the large-diameter portion and the two branch portions;
wherein, when the sum of the diameters of the two branch portions is smaller than the diameter of the large-diameter portion, the large-diameter portion has a 1/1 plain woven structure; the two branch portions have a 1/2 rib woven structure, or have a 1/1 plain-1/2 rib one-yarn-alternating or two-yarn-alternating woven structure; the tapered portion is bounded, in one front view, by a line in contact with the large-diameter portion, and a line in contact with the two branch portions; and shifting from the woven structure of the large-diameter portion to the woven structure of the two branch portions occurs to form a triangular shape

from the foot of the line extending, from the meeting point of the two branch portions on the line in contact with the two branch portions, toward, and perpendicularly crossing with, the line in contact with the large-diameter portion,
toward two arbitrary points on the line in contact with the two branch portions; and
wherein, when the sum of the diameters of the two branch portions is larger than the diameter of the large-diameter portion, the large-diameter portion has a 1/2 rib woven structure, or has a 1/1 plain-1/2 rib one-yarn-alternating or two-yarn-alternating woven structure; the two branch portions have a 1/1 plain woven structure; the tapered portion is bounded, in one front view, by a line in contact with the large diameter portion, and a line in contact with the two branch portions; and shifting from the woven structure of the two branch portions to the woven structure of the large-diameter portion occurs to form a triangular shape
from the meeting point of the two branch portions on the line in contact with the two branch portions
toward two arbitrary points on the line in contact with the large-diameter portion;

(3) the woven fabric in each of the large-diameter portion and the two branch portions has a cover factor of 1600 to 2400; and
(4) the thickness of the woven fabric in each of the large-diameter portion and the two branch portions is not more than 110 $\mu$m.

**2.** The medical high density woven fabric according to claim 1, wherein the woven fabric in the tapered portion also has a cover factor of 1600 to 2400.

**3.** The medical high density woven fabric according to claim 2, wherein the woven fabric in each of the large-diameter portion, the two branch portions, and the tapered portion has a water permeability of not more than 500 ml/cm$^2$/min.

**4.** The medical high density woven fabric according to any one of claims 1 to 3, wherein the weft yarn is a synthetic polyester multifilament fiber having a monofilament fineness of not more than 0.5 dtex.

**5.** The medical high density woven fabric according to any one of claims 1 to 4, wherein the woven fabric in each of the large-diameter portion, the two branch portions, and the tapered portion has a burst strength of not less than 100 N.

# FIG. 1

Large-diameter portion (A)

Tapered portion

Branch portions (B)

4

1

2

3

1/1 Plain

1/2 Rib

The gray portions in the left panel have the 1/1 plain-1/2 rib combination illustrated above (one-yarn-alternating structure)

# FIG. 2

1/1 Plain

1/2 Rib

Large-diameter portion (A)

Tapered portion

1

2

3

Branch portions (B)

The gray portions in the left panel have the 1/1 plain-1/2 rib combination illustrated above (one-yarn-alternating structure)

# FIG. 3

1/1 Plain structure

1/1 Plain structure 3D

# FIG. 4

1/1 Plain double-woven structure
(basic structure)

1/1 Plain double-woven structure 3D

# FIG. 5

1/2 Rib woven structure

1/2 Rib woven structure 3D

# FIG. 6

1/2 Rib double-woven structure (basic structure)

1/2 Rib double-woven structure 3D

# FIG. 7

1/1 Plain-1/2 rib
one-yarn-alternating woven
structure

1/1 Plain-1/2 rib
one-yarn-alternating woven
structure 3D

# FIG. 8

1/1 Plain-1/2 rib
one-yarn-alternating double-woven
structure (basic structure)

1/1 Plain-1/2 rib
one-yarn-alternating double-woven
structure 3D

# FIG. 9

1/1 Plain-1/2 rib
two-yarn-alternating woven
structure

1/1 Plain-1/2 rib
two-yarn-alternating woven structure 3D

# FIG. 10

1/1 Plain-1/2 rib
two-yarn-alternating double-woven structure 3D

1/1 Plain-1/2 rib
two-yarn-alternating double-woven structure
(basic structure)

FIG. 11

**INTERNATIONAL SEARCH REPORT**

International application No.

PCT/JP2018/045747

A. CLASSIFICATION OF SUBJECT MATTER

Int.Cl.   A61F2/07(2013.01)i,   A61L31/06(2006.01)i,   A61L31/12(2006.01)i,
          A61L31/14(2006.01)i, D03D1/00(2006.01)i, D03D3/02(2006.01)i

According to International Patent Classification (IPC) or to both national classification and IPC

B. FIELDS SEARCHED

Minimum documentation searched (classification system followed by classification symbols)

Int.Cl.   A61F2/06-A61F2/07,  A61L31/06,  A61L31/12,  A61L31/14,  D03D1/00,
          D03D3/02

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2017/126009 A1 (ASAHI KASEI CORPORATION) 27 July 2017 & US 2019/0015192 A1 & CN 108472122 A & KR 10-2018-0094001 A | 1–5 |
| A | JP 2016-123764 A (ASAHI KASEI CORPORATION) 11 July 2016 (Family: none) | 1–5 |
| A | US 2009/0171450 A1 (AESCULAP AG) 02 July 2009 & EP 2074960 A1 & DE 102007063265 A1 & ES 2440724 T3 & EP 2074960 B1 & US 08728151 B2 | 1–5 |
| A | US 2005/0240261 A1 (SCIMED LIFE SYSTEMS, INC.) 27 October 2005 & WO 2005/115272 A2 & CA 2561900 A1 & US 2010/0137969 A1 & US 2013/0116768 A1 & US 07682381 B2 & US 08343207 B2 | 1–5 |

☐ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 February 2019 (21.02.2019) | 05 March 2019 (05.03.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

30

**EP 3 733 122 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013137263 A
- JP 2016123764 A
- JP 4540912 B